# FASCICULE DE BREVET EUROPEEN

(11) **EP 2 493 397 B1**
(45) Date de publication et mention de la délivrance du brevet: **16.12.2015**
(21) Numéro de dépôt: 10776967.1
(22) Date de dépôt: 26.10.2010
(51) Int. Cl.: A61B 17/16, A61F 2/30

(54) **Arbre d'entraînement pour alésoir chirurgical**
Antriebswelle für einen chirurgischen Fräser
Drive shaft for a surgical reamer

(30) Priorité: 28.10.2009 CH 16572009
(43) Date de publication de la demande: 05.09.2012
(73) Titulaire: Chirmat Sàrl, 1870 Monthey (CH)
(72) Inventeur: ARLETTAZ, Yvan, CH-1870 Monthey (CH); BONJOUR, Christian, CH-1268 Begnins (CH)
(74) Mandataire: P&TS SA (AG, Ltd.)
(86) Numéro de dépôt international: PCT/EP2010/066120
(87) Numéro de publication internationale: WO 2011/051260

(56) Documents cités:
- WO-A1-2006/046789
- WO-A1-2009/015672

## Description

### Domaine technique

La présente invention concerne un arbre d'entraînement pour un alésoir chirurgical, en particulier un arbre d'entraînement avec une structure en matériau composite renfoncée par des fibres longues et/ou courtes pour entraîner une tête d'alésage interchangeable pour aléser le canal intramédullaire.

### Etat de la technique

L'enclouage centromédullaire est une des techniques thérapeutiques les plus utilisées dans le traitement des fractures et dans la reconstruction des os longs, par exemple et de façon pas limitative le fémur, mais aussi le tibia ou l'humérus. Selon cette méthode, on élargit le canal médullaire au diamètre souhaité avec plusieurs têtes interchangeables d'alésage de différent diamètre montées successivement sur un arbre d'entraînement souple, pour suivre la courbure du canal médullaire.

On connaît des arbres d'entraînement métalliques avec une structure hélicoïdale assurant la flexibilité axiale et la rigidité à la torsion souhaités. Ces dispositifs sont toutefois coûteux et doivent être réutilisés. Ils sont cependant difficiles à nettoyer et à stériliser, particulièrement en milieu hospitalier.

Le brevet US7407440 divulgue des arbres métalliques tubulaires à paroi mince en matériaux super-élastiques, par exemple en nitinol. Si ces arbres sont plus faciles à nettoyer et stériliser, ils sont toutefois plus coûteux et fragiles que les arbres d'entraînement conventionnels.

WO2006/046789 décrit un alésoir utilisé dans le domaine des machines industrielles, par exemple pour réaliser un trou dans une came d'un moteur d'un véhicule, qui comprend un corps composé par une première pièce en fibre de carbone qui est contenue dans une deuxième pièce métallique, composée par une ou plusieurs parties. La deuxième pièce protège la première et donne au dispositif une meilleure rigidité. Un adaptateur inséré dans la première pièce est destiné à coopérer à travers une vis avec une tête d'alésage interchangeable. Une tige centrale métallique est insérée dans la première pièce : son diamètre est lié à la fréquence de résonance du dispositif. Du liquide réfrigérant peut circuler dans le dispositif quand il est utilisé à des vitesses élevées. Le dispositif de cette demande n'est pas destiné à aléser un os, le but étant d'augmenter la rigidité du dispositif et d'aléser des trous à haute vitesse sans créer des effets de vibration.

WO2009/015672 concerne un arbre de transmission en fibre de carbone, et des éléments de connexion pour le manche et l'alésoir. Ce dispositif n'est pas réalisé en fibre de verre et possède un système de raccord distal compliqué.

### Bref résumé de l'invention

Un but de la présente invention est de proposer un arbre d'entraînement pour l'alésage du canal médullaire des os longs plus économique et fiable que les dispositifs connus.

Un autre but est de proposer un arbre d'entraînement pour alésoir chirurgical qui soit facile et possible à stériliser en milieu industriel lors de sa fabrication, mais qui soit suffisamment économique pour pouvoir être jeté après le premier usage afin d'éviter une stérilisation en milieu hospitalier, plus coûteuse.

Un autre but de la présente invention est de proposer un arbre d'entraînement qui ne présente pas les difficultés de nettoyage et stérilisation présentés par les dispositifs de l'art antérieur.

Selon l'invention, ces buts sont atteints notamment au moyen du dispositif qui est l'objet de la revendication principale, et notamment par un arbre d'entraînement pour alésoir chirurgical comprenant une tige ayant une première extrémité et une deuxième extrémité, un élément de connexion pour l'engagement d'une tête d'alésage interchangeable, dans lequel la tige allongée a une structure composite comprenant des fibres, et dans lequel une portion dudit élément de connexion est insérée dans une cavité de ladite première extrémité de ladite tige.

Cette solution présente notamment l'avantage que l'arbre d'entraînement ainsi obtenu est plus léger et moins cher que les arbres d'entraînement de l'art antérieur, et peut être utilisé une seule fois et détruit après l'usage, évitant ainsi les difficultés et les coûts de stérilisation liés à l'usage des arbres d'entraînement connus.

Cette solution permet aussi de réaliser un arbre flexible avec un élément de connexion métallique à son extrémité et une section transversale constante le long de la tige et de l'élément de connexion, simplifiant aussi l'insertion et la rétraction de l'outil. Cet arbre est creux pour permettre le passage d'une tige de Kirschner comme référence.

### Brève description des figures

Des exemples de mise en oeuvre de l'invention sont indiqués dans la description illustrée par les figures annexées dans lesquelles :
La figure 1 illustre un mode de réalisation de l'invention.
La figure 2 montre en coupe une extrémité de l'arbre de transmission de la figure 1.
Les figures 3a à 3c montrent respectivement une vue de coupe, par le haut et latérale d'un mode de réalisation de l'invention.
Les figures 4a à 4b illustrent respectivement une vue de section et latérale d'une extrémité d'un mode de réalisation de l'invention.

### Exemple(s) de mode de réalisation de l'invention

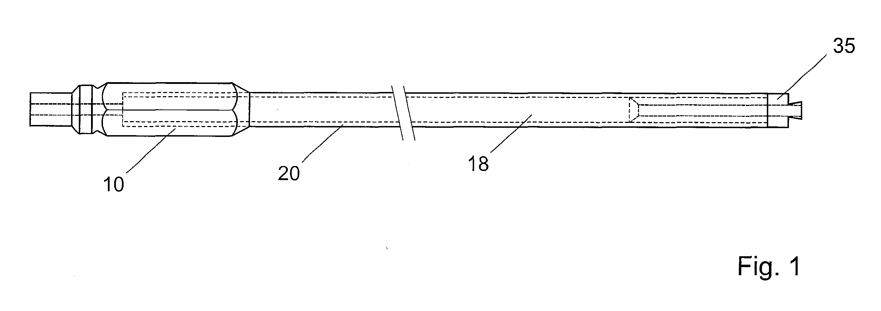

En référence à la figure 1, selon un mode de réalisation, l'arbre de transmission de l'invention comporte une tige souple 20 en un matériau composite, par exemple avec une matrice plastique renforcée par des fibres. La longueur de la tige est adaptée à celle du canal médullaire de l'os sur lequel on veut intervenir. Dans une variante préférentielle cette longueur supérieure à 70 cm, par exemple 1 m, ce qui permet avantageusement de faire de l'arthrodèse en une seule fois. Les arbres de transmission métalliques avec une spire bobinée connus ont une longueur d'environ 50 cm et donc ils ne permettent pas de faire de l'arthrodèse en une seule fois. En plus il existe des difficultés de réalisation d'un arbre de transmission métallique connu ayant une longueur inférieure à 1 m, obtenu par coupage d'un arbre de plus grande longueur, à cause de la structure hélicoïdale du ressort.

Dans une variante préférentielle l'arbre de l'invention est fabriqué avec une longueur de 1 m. Puisqu'il est réalisé en un matériau composite, il est possible de le couper en deux parties égales pour avoir deux arbres de longueur 50 m. De la même façon, il est possible de le couper en deux parties qui ne sont pas égales et donc obtenir deux arbres avec une longueur différente. En d'autres termes il n'y a pas deux procédés de fabrication différents pour réaliser un arbre de longueur 1 m et un arbre de longueur inférieure, par exemple de 50 cm, puisque l'arbre de longueur inférieure est obtenu à partir de l'arbre de longueur 1 m. Réaliser un arbre métallique connu avec une longueur de 1 m, même s'il n'y avait pas les difficultés techniques mentionnées, requerrait une production spécifique et donc coûteuse.

Dans le cadre de l'invention, on peut utiliser différentes fibres, selon les caractéristiques mécaniques et économiques que l'on recherche, par exemple des fibres de verre, des fibres de carbone ou de fibres d'aramide. Les fibres sont préférablement englobées dans une matrice polymère en résine époxyde, polyester, ester vinylique, phénolique, polyimide, polyamide, polypropylène, polyétheréthercétone (PEEK), ou tout autre matériau approprié.

Préférablement, la tension et l'orientation des fibres de la tige 20 sont prédéterminées et contrôlées de manière à obtenir une tige ayant les caractéristiques de souplesse en flexion et de résistance à la torsion requises. Selon un aspect de l'invention, plusieurs couches superposées de fibres sont bobinées en tension sur un mandrin cylindrique. La résine peut être appliquée avant ou après le bobinage. L'orientation des fibres par rapport à l'axe de la tige et leur tension sont choisies de manière à obtenir les caractéristiques mécaniques souhaitées. Des essais concluants ont été effectués avec un tressage comportant simultanément des fibres orientées à 45°, 90° et 135° par rapport à l'axe longitudinal de l'arbre..

Selon un autre aspect de l'invention, la tige 20 est obtenue à partir d'un tissu de fibres longues (> 10 cm) tressées, ou de rubans tissés, enrobés dans une matrice de la résine choisie et enroulé sur un mandrin. La structure composite est ainsi réalisée à partir de tissus pre-imprégnés. Dans une autre variante il est possible d'injecter un tel arbre avec un résine chargée de fibres courtes (< 10 cm) en verre ou en carbone pour réaliser des arbres résistent à des plus faibles efforts et ayant un coût faible. Dans ces cas également, les fibres ont préférablement des orientations prédéterminées par rapport à l'axe de la tige.

Dans une variante préférentielle la tige 20 est dotée d'un élément, tel qu'une bande, ou un fil ou une poudre radio-opaque, ce qui permet de la visualiser radiologiquement lors de l'intervention.

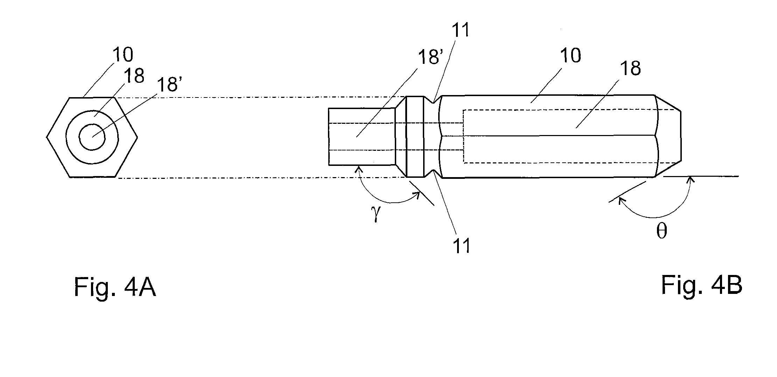

La tige 20 comporte un élément d'interface 10 à une extrémité pour la connexion à un moteur, une perceuse, ou un manche, permettant au chirurgien de guider et mettre en rotation la tête d'alésage. Dans l'exemple illustré sur les figures 1, 4a et 4b, l'interface 10 a une section hexagonale standardisée, visible notamment sur la figure 4b. La valeur du premier angle γ et du deuxième angle θ dans la variante de la figure 4b sont respectivement de 135° et 150°. D'autres structures sont toutefois possibles.

L'interface 10 présente, comme détaillé sur la Figure 4a, un canal axial 18 qui traverse l'arbre d'entraînement d'une extrémité à l'autre et aussi un canal 18', de diamètre inférieure au diamètre du canal 18, pour l'insertion de la tige de Kirschner. Comme illustré sur la Figure 4b, le raccord entre le canal 18' et le canal 18 complique le nettoyage du dispositif.

La gorge 11 a une géométrie adaptée à chaque type de perceuse : la figure 4b illustre un exemple d'une telle géométrie.

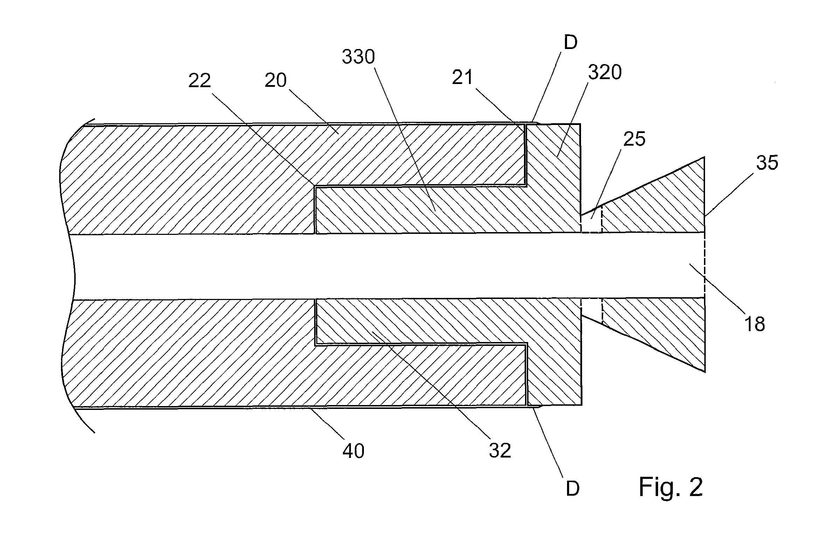

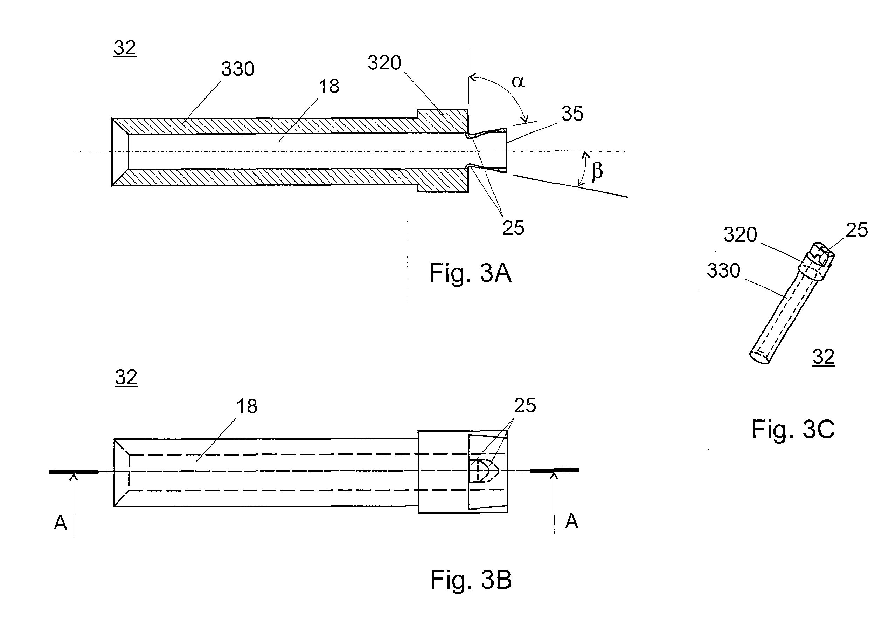

L'élément de connexion 35 est connecté à une première extrémité de la tige 20 et permet l'engagement d'outils interchangeables, par exemple une tête d'alésage, un taraud, ou tout autre outil orthopédique. Dans l'exemple illustré sur les figures 2 à 3c, l'élément de connexion 35 comporte un tenon en queue d'aronde, destiné à s'engager dans une rainure à section trapézoïdale de la tête d'alésage. Le tenon à queue d'aronde est seulement un possible exemple de l'élément de connexion et d'autres éléments de connexion peuvent être utilisés.

Si la tête d'alésage ne présente pas un élément de connexion, par exemple une queue d'aronde, ayant une géométrie compatible avec celle de l'élément de connexion 35 de l'arbre, il est possible d'utiliser un dispositif d'accouplement mécanique intermédiaire pour relier la tête d'alésage à l'arbre. Ce dispositif d'accouplement peut être externe et/ou interne. Les accouplements externes peuvent être par exemple du type queue d'aronde, pivot glissant, baïonnette, en T, à pince extensible externe, etc. Les accouplements internes peuvent être par exemple du type pince extensible interne, à arbre cannelé, à profil polygone, à clavette libre, etc.

Un trou transversal 25 permet de sécuriser l'outil sur l'arbre au moyen d'une goupille ou d'une vis. D'autres moyens de fixation sont toutefois possibles. Toutefois la présence d'une tige de Kirschner suffit en général à sécuriser l'outil.

En raison des efforts mécaniques et de l'usinage délicat auquel il doit être soumis, l'élément de connexion 35 est préférablement réalisé en acier chirurgical inoxydable ou dans un autre matériau métallique biocompatible. L'élément de connexion 35 comporte une portion 32 de dimension transversale réduite, mieux visible sur les figures 2 à 3c, insérée dans un dégagement à la première extrémité de la tige 20 en composite. Cette portion 32 dans une variante comporte une première partie 320 ayant un diamètre égale au diamètre externe de la tige 20 et une deuxième partie 330 ayant un diamètre réduit semblable au diamètre sur l'élément de connexion 35. Dans la variante illustrée sur la figure 3a, l'angle qui définit l'ouverture de l'élément 35 est égal à 2*β. Dans la variante illustrée β = 10°. Sur la même figure l'angle entre l'élément 35 et la partie 320 de la portion 32 est indiqué avec la référence α. Dans la variante illustrée α = 80°.

Préférablement la tige 20, l'élément de connexion 35 et l'élément d'interface 10 présentent un canal axial 18 traversant l'arbre d'entraînement d'une extrémité à l'autre. Cette caractéristique permet de glisser l'arbre de l'invention sur une tige de Kirschner préalablement insérée dans le canal médullaire.

Préférablement le diamètre transversal externe de l'élément de connexion 35 est égal au diamètre transversal externe de la tige 20, donnant ainsi un arbre d'entraînement de section constante, de son extrémité jusqu'à l'élément d'interface 10, et dont la surface extérieure est parfaitement lisse. Cette structure assure une insertion et un retrait de l'arbre d'entraînement faciles, et minimise le risque de blocage de l'outil dans la cavité médullaire ; elle facilite en outre la stérilisation. En tout cas le dispositif n'est pas été conçu pour pouvoir être stérilisé. Dans une autre variante, par exemple illustrée sur les figures 1 et 3a, le diamètre transversal externe de l'élément de connexion 35 est inférieur au diamètre transversal externe de la tige 20.

Dans une variante le diamètre transversal externe de la tige 20 est de 8 mm environ, pour des os tels que le fémur. Dans une autre variante ce diamètre est de 5-6 mm environ, pour des os plus petits, tels que le tibia ou tout autre os long. Comme discuté, le diamètre de l'élément de connexion 35 a un diamètre qui s'adapte à celui externe de la tige 20.

De la même manière, le diamètre interne du canal axial 18 à travers l'élément de connexion est égal au diamètre interne du canal axial à travers la tige 20. On assure ainsi une surface du canal lisse, ce qui facilite l'insertion de l'arbre autour d'une tige de guidage pré-enfilée dans l'os.

Dans une variante non représentée, le canal interne de la tige a un diamètre constant et est dépourvu de dégagement pour l'élément de connexion 35, qui forme dans ce cas une saillie limitant le diamètre interne du canal. Cette solution est moins coûteuse, mais rend l'insertion de l'alésoir sur la tige de guidage un peu plus difficile.

Plusieurs essais ont montré que la liaison entre l'élément de connexion 35 et la tige 20, et même l'ensemble de la tige, sont plus solides si cette dernière est réalisée en résine renforcée de fibre de verre plutôt qu'en fibre de carbone par exemple. Une possible explication de ce phénomène inattendu est que les tiges réalisées à partir de fibre de carbone ou d'autres fibres à haute performance doivent avoir une épaisseur de paroi très réduite pour offrir la souplesse requise afin de suivre la courbure de la cavité médullaire. Une tige à paroi très mince en fibre de carbone est certes résistante en flexion, mais résiste en revanche mal à l'écrasement. Par ailleurs, les parois très minces qui seraient nécessaires en cas de fabrication en fibre de carbone ne permettent pas d'y insérer un élément de connexion 35 avec un diamètre de canal 18 égal à celui du canal axial dans la tige. On privilégiera l'utilisation d'une fibre de verre.

La liaison entre l'élément de connexion 35 et la tige allongée 20 peut être améliorée en structurant les surfaces de la portion à dimension transversale réduite 32 et/ou la surface interne de la cavité dans laquelle elle est logée par des nervures, des protubérances et des cavités. Préférablement, la portion à dimension transversale réduite 32 est maintenue en son logement par une couche d'adhésif 22, par exemple un adhésif thermofusible, ou un adhésif époxyde, ou tout autre adhésif ayant une excellente adhésion au métal de l'élément de connexion 35 et à la résine de la matrice de la tige 20. Dans une variante cet adhésif est une colle fusible, de façon à ce que, si l'arbre était porté à des températures élevées, par exemple lors d'une stérilisation, cette colle perdrait ses caractéristiques mécaniques et l'arbre serait inutilisable. La présence de cette colle en d'autres termes évite un deuxième usage de l'arbre qui, donc, est dans ce cas à usage unique. Cet adhésif est aussi présent entre la tige 20 et l'élément d'interface 10. Eventuellement, la portion à dimension réduite 32 et son logement peuvent avoir une section non ronde, par exemple carrée, afin de mieux transmettre le couple de rotation à l'outil.

Selon un autre aspect de l'invention, la portion à dimension transversale réduite 32 est maintenue en son logement par des moyens de fixation tels que des vis, des rivets ou des languettes. Ces moyens de fixation peuvent être employés seuls, ou en combinaison avec un adhésif comme mentionné ci-dessus. Préférablement les têtes des vis ou des rivets sont abrités dans des chanfreins, de manière à ne pas dépasser au-delà de la surface extérieure lisse de la tige 20. Selon un autre aspect préférentiel de l'invention, au moins une partie de la tige 20 est couverte avec un film biocompatible 40. La surface interne du canal 18 peut aussi être couverte de la même manière. Comme illustré par les détails D de la figure 2, ce film 40 couvre aussi une partie de la portion de l'élément de connexion 32 pour garantir l'étanchéité du dispositif.

La présente invention permet de réaliser des arbres d'entraînement souples et résistants, à un coût de fabrication réduit. Ces arbres peuvent avantageusement être employés comme outils à usage unique dans les opérations de pose de clous centromédullaires, éliminant ainsi tous les problèmes de nettoyage et stérilisation des arbres d'entraînement connus.

### Numéros de référence employés sur les figures

- 10: Elément d'interface
- 11: Gorge de l'élément d'interface
- 20: Tige
- 18: Canal axial
- 18': Canal pour l'insertion de la tige de Kirschner
- 21: Première extrémité de la tige
- 22: Adhésif
- 25: Trou transversal
- 32: Portion de l'élément de connexion
- 320: Première partie de la portion de l'élément de connexion
- 330: Deuxième partie de la portion de l'élément de connexion
- 35: Elément de connexion
- 40: Film biocompatible
- α: Angle entre l'élément de connexion 35 et la première partie 320
- β: Semi-ouverture de l'élément de connexion 35
- γ: Premier angle de l'élément d'interface 10
- θ: Deuxième angle de l'élément d'interface 10

## Revendications

1. Arbre d'entraînement pour alésoir chirurgical comprenant une tige (20) ayant une première extrémité (21) et une deuxième extrémité, un élément de connexion (35) pour l'engagement d'une tête d'alésage interchangeable, dans lequel la tige allongée (20) a une structure composite comprenant des fibres,
**caractérisé en ce qu'** une portion (32) dudit élément de connexion (35) est insérée à l'intérieur d'une cavité de la première extrémité (21) de ladite tige (20).

2. Arbre d'entraînement selon la revendication précédente, dans lequel le diamètre transversal dudit élément de connexion est égal au diamètre transversal de ladite tige (20).

3. Arbre d'entraînement selon l'une des revendications précédentes ayant un diamètre transversal essentiellement constant.

4. Arbre d'entraînement selon l'une des revendications 2 ou 3, dans lequel ledit diamètre transversal est compris dans la plage 5-8 mm.

5. Arbre d'entraînement selon l'une des revendications précédentes, dans lequel ladite structure composite comprend des fibres de verre.

6. Arbre d'entraînement selon l'une des revendications précédentes, dans lequel ladite structure composite comprend des fibres bobinées.

7. Arbre d'entraînement selon l'une des revendications 1 à 5, dans lequel ladite structure composite est réalisée à partir de tissus pre-imprégnés.

8. Arbre d'entraînement selon l'une des revendications précédentes, dans lequel ladite tige (20) est au moins partiellement recouverte par un film (40) biocompatible.

9. Arbre d'entraînement selon l'une des revendications précédentes, dans lequel ladite tige (20) et ledit élément de connexion présentent un canal axial (18) et dans lequel le diamètre dudit canal axial (18) est essentiellement constant.

10. Arbre d'entraînement selon l'une des revendications précédentes, dans lequel ledit élément de connexion comporte un tenon en queue d'aronde (35) destiné à s'engager dans une rainure à section trapézoïdale d'une tête d'alésage et/ou dans un dispositif d'accouplement mécanique intermédiaire.

11. Arbre d'entraînement selon l'une des revendications précédentes, dans lequel ladite portion (32) de l'élément de connexion (35) insérée dans une cavité (21) de la dite tige (20) est maintenue par un adhésif (22).

12. Arbre d'entraînement selon la revendication 11, dans lequel ledit adhésif (22) est une colle fusible.

13. Arbre d'entraînement selon l'une des revendications précédentes, dans lequel ladite portion (32) de l'élément de connexion (35) insérée dans une cavité (21) de la dite tige (20) est maintenue par des moyens de fixations tels que rivets, vis ou languettes.

14. Arbre d'entraînement selon l'une des revendications précédentes, dans lequel la seconde extrémité de ladite tige (20) porte un élément d'interface (10) connectable avec une perceuse ou avec un manche.

15. Arbre d'entraînement selon l'une des revendications précédentes, comprenant un élément radio-opaque.

## Patentansprüche

1. Antriebswelle für einen chirurgischen Fräser, umfassend einen Schaft (20) mit einer ersten Extremität (21) und einer zweiten Extremität, ein Verbindungselement (35) zum Einrasten eines auswechselbaren Fräserkopfes, worin der längliche Schaft (20) eine Verbundstruktur mit Fasern aufweist,
**dadurch gekennzeichnet, dass** eine Portion (32) des besagten Verbindungselements (35) in einen Hohlraum der ersten Extremität (21) des besagten Schafts (20) eingeführt wird.

2. Antriebswelle gemäss dem vorhergehenden Anspruch, worin der Querschnittsdurchmesser des besagten Verbindungselements gleich dem Querschnittsdurchmesser des besagten Schafts (20) ist.

3. Antriebswelle gemäss einem der vorhergehenden Ansprüche, mit einem Querschnittsdurchmesser, welcher im Wesentlichen konstant ist.

4. Antriebswelle gemäss einem der Ansprüche 2 bis 3, worin der besagte Querschnittsdurchmesser im Bereich zwischen 5-8mm liegt.

5. Antriebswelle gemäss einem der vorhergehenden Ansprüche, worin die besagte Verbundstruktur Glasfasern umfasst.

6. Antriebswelle gemäss einem der vorhergehenden Ansprüche, worin die besagte Verbundstruktur gewickelte Fasern umfasst.

7. Antriebswelle gemäss einem der Ansprüche 1 bis 5, worin die besagte Verbundstruktur aus vorimprägnierten Geweben hergestellt wird.

8. Antriebswelle gemäss einem der vorhergehenden Ansprüche, worin der besagte Schaft (20) zumindest teilweise durch einen biokompatiblen Film (40) bedeckt ist.

9. Antriebswelle gemäss einem der vorhergehenden Ansprüche, worin der besagte Schaft (20) und das besagte Verbindungselement einen axialen Kanal (18) aufweisen und worin der Durchmesser des besagten axialen Kanals (18) im Wesentlichen konstant ist.

10. Antriebswelle gemäss einem der vorhergehenden Ansprüche, worin das besagte Verbindungselement einen Schwalbenschwanzzapfen (35) aufweist, welcher dazu bestimmt ist, in eine Rille mit trapezförmigem Querschnitt eines Fräserkopfes und/oder in eine mechanische Zwischenkupplungsvorrichtung einzugreifen.

11. Antriebswelle gemäss einem der vorhergehenden Ansprüche, worin die besagte Portion (32) des in einen Hohlraum (21) des besagten Schafts (20) eingeführten Verbindungselements (35) durch ein Klebemittel (22) gehalten wird.

12. Antriebswelle gemäss Anspruch 11, worin das besagte Klebemittel (22) ein Haftschmelzkleber ist.

13. Antriebswelle gemäss einem der vorhergehenden Ansprüche, worin die besagte Portion (32) des in einen Hohlraum (21) des besagten Schafts (20) eingeführten Verbindungselements (35) durch Befestigungsmittel wie Nieten, Schrauben oder Laschen gehalten wird.

14. Antriebswelle gemäss einem der vorhergehenden Ansprüche, worin die zweite Extremität des besagten Schafts (20) ein mit einem Bohrer oder mit einem Griff verbindbares Schnittstellenelement (10) trägt.

15. Antriebswelle gemäss einem der vorhergehenden Ansprüche, mit einem strahlungsundurchlässigen Element.

## Claims

1. Drive shaft for a surgical reamer comprising a rod (20) having a first extremity (21) and a second extremity, a connecting element (35) for engaging an interchangeable reamer head, wherein the elongated rod (20) has a composite structure comprising fibres,
**characterised in that** a portion (32) of said connecting element (35) is inserted inside a cavity of the first extremity (21) of said rod (20).

2. Drive shaft according to the preceding claim, wherein the transverse diameter of said connecting element is equal to the transverse diameter of said rod (20).

3. Drive shaft according to one of the preceding claims, having a transverse diameter that is essentially constant.

4. Drive shaft according to one of the claims 2 or 3, wherein said transverse diameter is comprised in the range 5-8 mm.

5. Drive shaft according to one of the preceding claims, wherein said composite structure comprises glass fibres.

6. Drive shaft according to one of the preceding claims, wherein said composite structure comprises wound fibres.

7. Drive shaft according to one of the claims 1 to 5, wherein said composite structure is made from pre-impregnated fabrics.

8. Drive shaft according to one of the preceding claims, wherein said rod (20) is at least partly covered by a biocompatible film (40).

9. Drive shaft according to one of the preceding claims, wherein said rod (20) and said connecting element have an axial bore (18) and wherein the diameter of said axial bore (18) is essentially constant.

10. Drive shaft according to one of the preceding claims, wherein said connecting element comprises a dovetail tenon (35) designed to engage in a groove having a trapezoidal cross-section of a reamer head and/or in an intermediary mechanical coupling device.

11. Drive shaft according to one of the preceding claims, wherein said portion (32) of the connecting element (35) inserted in a cavity (21) of said rod (20) is held by an adhesive (22).

12. Drive shaft according to claim 11, wherein said adhesive (22) is a hot-melt glue.

13. Drive shaft according to one of the preceding claims, wherein said portion (32) of the connecting element (35) inserted in a cavity (21) of said rod (20) is held by fastening means such as rivets, screws or tabs.

14. Drive shaft according to one of the preceding claims, wherein the second extremity of said rod (20) bears an interface element (10) connectable with a drill or with a handle.

15. Drive shaft according to one of the preceding claims, comprising a radio-opaque element.
